# EUROPEAN PATENT APPLICATION

(11) **EP 1 658 898 A1**
(43) Date of publication of application: **24.05.2006**
(21) Application number: 04027624.8
(22) Date of filing: 20.11.2004
(51) Int. Cl.: B01L 7/00, C12Q 1/68

(54) **Nucleic acid preparation**

(71) Applicant: Roche Diagnostics GmbH, 68305 Mannheim (DE); F. HOFFMANN-LA ROCHE AG, 4070 Basel (CH)
(72) Inventor: Kopp, Martin, Dr., 6332 Hagendorn (CH)
(74) Representative: Knauer, Martin

(57) **Abstract**

The present invention is directed to methods, devices and computer programs for preparing nucleic acids from a template nucleic acid by subjecting a sample to thermocycles. After a first number of thermocycles, a partial amount of the reaction mixture is being subjected to a second number of thermocycles. This two step amplification method allows to speed up overall reaction time without affecting the limit of detection.

## Description

### Field of the invention

The present invention is related to a method of preparing nucleic acids from a template nucleic acid, a diagnostic device for preparing nucleic acids from a template, a computer program for controlling a method for the preparation of nucleic acids from a template nucleic acid using thermocycles, a computer program product comprising said program, an apparatus for preparing nucleic acids and a method for determining the presence or absence or amount of a template nucleic acid in a sample.

### Background of the invention

Methods for amplification of nucleic acids from samples containing these nucleic acids are known. In in-vivo methods, micro-organisms with a genome genetically engineered to contain the nucleic acid to be amplified are used to produce large amounts of copies of the nucleic acid. Those methods are slow and require a lot of experimentation before successful implementation. More recently, in-vitro methods have been established to prepare large amounts of nucleic acids without the involvement of micro-organisms. The first in-vitro amplification method was Polymerase Chain Reaction (PCR), described in EP 201 184. In a very preferred embodiment of PCR, the sample containing the nucleic acid to be amplified is repeatedly subjected to a temperature profile reflecting the steps of primer hybridization to the target nucleic acid, elongation of said primer to prepare an extension product using the nucleic acid to be copied as a template and separating the extension product form the template nucleic acid. The temperature profile is applied several times, allowing the repetition of the steps, including hybridization and elongation of a second primer capable of hybridizing to the extension product of the first primer. Each repeatedly performed temperature profile is called a thermocycle.

This method has been applied to methods for the determination of nucleic acids based on the superior sensitivity of detection provided by the increased amount of nucleic acids. In EP 200 362 there is disclosed a method using adding a probe capable of hybridizing to the nucleic acids formed in the reaction mixture and detecting the presence, absence or amount of hybrids formed as a measure of the original nucleic acid in the sample.

More recently, it has been found that methods for the amplification of nucleic acids are so effective that there is a danger of contamination of the environment, e.g. the laboratory in which the amplification reaction is performed. This may yield in false positive results of subsequent detections. In EP 543 942 there is disclosed a method which does not need opening of the reaction chamber, vessel or tube between amplification and detection of hybrids to add the probe. Those methods are called homogenous amplification and detection methods.

The time necessary for conducting an amplification reaction to a great extent depends on the reaction volume used. For example, when conducting a PCR reaction in a 50 - 100 µl volume on a thermocycler instrument as the PCR System 9700 instrument (Applied Biosystems), a reaction time of two to four hours is needed. Most of this time is needed for changing the temperature of the reaction mixture to conduct the thermocycles. This can be speed up by several means. Firstly, the shape of the reaction vessel can be changed to get an increased surface allowing a faster heating and cooling regime. Secondly, the reaction volume can be decreased so that less volume needs to be heated and cooled. By these means, thermocyclers like the LightCycler® (Roche Diagnostics) allow to decrease the reaction time up to several minutes instead of hours. However, the use of small reaction volumes has the disadvantage that also only small volumes of sample can be added to the reaction, which will proportionally reduce the limit of detection (LOD). Alternativelly the reaction volume could be maintained and the thermal diffusion distance could be minimised by large very flat amplification cell. However, this would lead to drastically increased amplification area and detection area and by these means very costly thermocycler and huge disposables. In addition the increased surface of such reaction chambers can inhibit the reaction.

In W02004/51218 there is disclosed a method for detecting different analytes wherein after a multiplex amplification of all ingredients of the reaction mixture the reaction mixture is split into aliquots and the aliquots be treated with reagents for specific amplification of specific analytes in separate reactions. This method has the disadvantage that it needs additional reagents for the second amplification.

In WO 02/20845 there is disclosed a method for avoiding primer-dimer formation by using a first amplification reaction with low primer concentration, then adding more primers and performing more amplification steps. Again, this method has the disadvantage that at a certain stage during amplification, the reaction tube must be opened to add more reagents. This is both inconvenient for the workflow in a laboratory and problematic for contamination reasons. In addition the use of a standard thermocycler does not allow very fast cycling speeds.

Both of the previously mentioned prior art documents do not aim to shorten the amplification time by any means, thus, it was the object of the present invention, to improve speed of amplification.

### Summary of the invention

In a first aspect, the invention is directed to a method of preparing nucleic acids from a template nucleic acid by subjecting a sample to thermocycles comprising
a) Subjecting a first amount of said sample in a first amplification chamber to a first number of thermocycles to prepare a first amount of a first reaction mixture, and
b) Subjecting a partial amount of said first reaction mixture in a second amplification chamber to a second number of thermocycles to prepare a second amount of a second reaction mixture
   wherein the volume of said second amplification chamber preferably is smaller than the volume of said first amplification chamber.

The integral heating and cooling speed preferably is at least 2 Kelvin/ second (K/s) in step a) and higher in step b), preferably at least 5 K/s.

In a second aspect, the invention is directed to a diagnostic device for preparing nucleic acids from a template comprising
- a first amplification chamber, and
- a second amplification chamber,
wherein the volume of said second amplification chamber preferably is smaller than the volume of said first amplification chamber. The integral heating and cooling speed preferably is at least 2 Kelvin/ second (K/s) in step a) and higher in step b), preferably at least 5 K/s.

In a third aspect, the invention is directed to a computer program for controlling a method for the preparation of nucleic acids from a template nucleic acid using thermocycles, characterized in that the computer program is set to apply a first number of thermocycles to the sample and subsequently a second number of thermocycles having a shorter cycling time on a different volume of a reaction mixture originating from the same sample.

In a fourth aspect, the invention is directed to a computer program product comprising said program on a physical storage means.

In a fifth aspect, the invention is directed to an apparatus for preparing nucleic acids comprising
- a thermocycler and
- a unit for controlling the thermocycler,
wherein the unit for controlling the thermocycler is loaded with said computer program.

In a sixth aspect, the invention is directed to a method for determining the presence or absence or amount of a template nucleic acid in a sample comprising the above described nucleic acids preparation method and detecting the formation of nucleic acids as a measure of the presence or absence or amount of nucleic acids to be determined.

### Brief description of the drawings

Fig. 1 illustrates the principle behind the present invention. By decreasing the reaction volume in a second step the required reaction time can be decreased without changing the limit of detection (LOD).
Fig. 2 shows a calculation of an optimized Aliquot-Amplification method.
Fig. 3 shows a thermocycling device comprising two amplification chambers useful for conducting the described nucleic acid preparation methods.
Fig. 4 shows a capillary disposable for conducting the methods of the present invention (see also Example 3).
Fig. 5 shows a possible device for conduction the described nucleic acid preparation methods in a multiplex fashion (see also Example 4).

### Detailed description of the invention

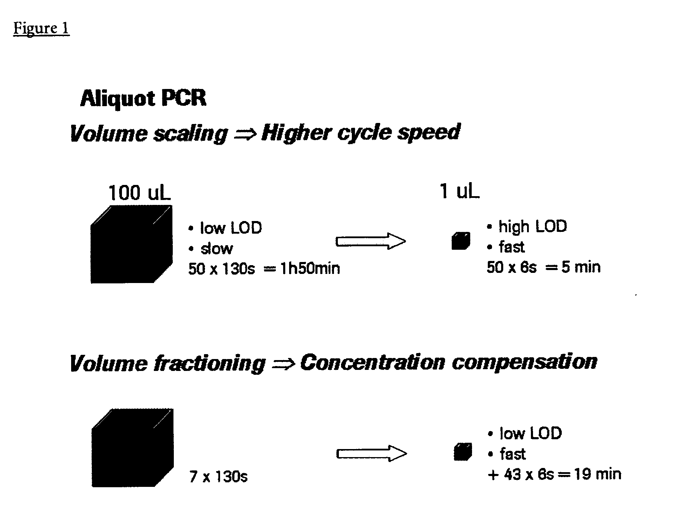

One aspect of the present invention is directed to a method of preparing nucleic acids from a template nucleic acid. In this method, a first amount of a sample is subjected to a first number of thermocycles to prepare a first amount of a first reaction mixture. An aliquot/ partial amount of that first reaction mixture is then subjected to a second number of thermocycles to prepare a second amount of a second reaction mixture. By subjecting only an aliquot of the first reaction mixture to a second number of thermocycles, the time per thermocycle can be decreased compared to the time necessary for thermocycling the first reaction mixture because of the reduced thermal diffusion distance. The first few thermal cycles are the most critical for the specificity of the amplification and need therefore very precise themperature levels without major over- respectively undershooting. Also a reaction volume of around 5 to 200 µl in the first reaction step provides sufficient volume to add enough of a nucleic acid preparation derived from a sample material to be analysed so that also very sensitive amplification methods are possible. The second part of additional 40-50 cycles is mainly needed to create a detectable signal level. According to these needs the cycler, the amplification chamber and the feedback control can be adjusted either to very accurate temperature levels or speed. In addition, the well confined, compact second amplification volume leads to a highly sensitive optical setup. This principle is being illustrated in Figure 1.

This method preferably is being based on the PCR-method, but also other methods can be used, such as linear or exponential nucleic acid amplification methods. Exponential amplification methods are well known in the art. Especially suitable are methods like PCR (US 4,683,202) and LCR (US 5,185,243, US 5,679,524 and US 5,573,907), in which the reaction mixture is repeatedly subjected to different temperatures (thermocycles).

The amount of sample, first and second number and length of thermocycles depend on the concrete purpose and amplification method used. The first amount of sample typically has a volume of 5 µl to 200 µl, preferably 5µl to 50µl. The further reagent necessary for conducting an amplification reaction can be added to the sample in dry form, for example as a deposit in the first amplification chamber, which deposit is solubilized by addition of the sample. These reagents can also be added in solution, typically in a volume of 2.5 to 100 µl, more preferably in a volume of 2.5 to 25 µl. The sample is then subjected in a first amplification chamber to a first number of thermocycles, which are typically 3 to 15 thermocycles, more preferably 5 to 8. The length of a thermocycle greatly varies between the different amplification methods. For PCR it typically varies between 20 seconds to 5 minutes, more preferably 20 to 120 seconds. In this step preferably an amplification chamber is used having an integral heating and cooling speed of at least 2 Kelvin/ second, more preferably between 4 to 7 K/s.

The integral heating respectively cooling speed can be described as the temperature step divided by the time needed to switch from one temperature level to the next temperature level. This is the relevant parameter in thermocycler instruments that can lead to faster PCR protocols. Typically these steps are from 95°C to 60°C, 60°C to 72°C and 72°C to 95°C. Therefore, in the context of the present invention integral heating and cooling speed is understood as the speed of a given amplification chamber and a given reaction volume in the temperature range of around 60°C and 95°C.
This integral heating and cooling speed is affected by the means used in the thermocycler for heating and cooling as well as by the size of the amplification chamber which determines the volume of the reaction mixture to be amplified. The use of a rapid thermocycler with an amplification chamber having a small volume allows short cycling times.
Conventional thermocyclers, based on Peltier technology (Applied Biosystem 9700) with a mounted aluminum block have typically integral ramping speeds smaller than 2-3 K/s and alone do therefore not allow taking full benefit of the herein proposed concept. With a thermocycler that yields a heating and cooling speed of 5-6 K/s like the LightCycler or instruments equipped with high performance Peltier elements first benefits could be seen. Even more benefit is achievable using thermocyclers that allow ramping speeds above 10 K/s in particular for the cooling rate.

A partial amount of said first amount of reaction mixture is then subjected in a second amplification chamber to a second number of thermocycles to prepare a second amount of a second reaction mixture. The volume of said partial amount of said first amount of reaction mixture typically has a volume of 00.5 to 5µL, more preferably 0.1-2 µL. Typically the partial amount of said reaction mixture is subjected to less than 50 thermocycles , more preferably between 20-40 thermocycles. The smaller volume of said partial amount of said first reaction mixture allows a higher integral heating and cooling speed of said second amplification chamber (at least 5 K/s, preferably between 8 to 12 K/s) and the length of a thermocycle can be less than the length of thermocycle in the first round of amplification and usually varies between 5 - 30 seconds.

The sample can be derived from human, animal and elsewhere in nature. Preferably samples, especially in diagnostic approaches, are blood, serum, plasma, bone marrow, tissue, sputum, pleural and peritoneal effusions and suspensions, urine, sperm and stool.

Preferably, the nucleic acids were purified from the samples prior to amplification, so that a more or less pure nucleic acid sample can be added to the amplification reaction. Methods for purifying nucleic acids are well known in the art. Beside laborious methods as described in Sambrook et al (Molecular Cloning - A Laboratory Manual, Coldspring Harbour Laboratory Press (1989)) also commercial kits are available for this purpose (for example, MagNAPure®, Roche Diagnostics).

Therefore the sample according to the present invention can be a sample directly derived from a donor, especially for cases where a further purification of the nucleic acids present in a sample is not needed as well as purified samples containing nucleic acids preparations from a donor sample.

Another aspect of the present invention is directed to a method for preparing and/ or detecting nucleic acids from a sample as described above in which the purification of the nucleic acids present in a sample is integrated preferably in the first amplification chamber of the device. Devices and methods in which the nucleic acids present in a sample are purified in the same reaction chamber as used for conducting a nucleic acid amplification reaction are known in the art. For example in WO 03/106031 integrated devices are described in which binding matrices like glass fleeces are used for capturing of nucleic acids present in a sample. Following the sample preparation the amplification reaction can be conducted in the same reaction chamber used for nucleic acid sample preparation. Such an approach can be combined with the methods and devices of the present invention. The nucleic acids of a sample can be purified and can be subjected to a first number of thermocycles to prepare a first amount of a first reaction mixture in a first amplification chamber of a device. An aliquot of said first reaction mixture can then be transferred to the second amplification chamber for the second number of thermocycles to prepare a second amount of a second reaction mixture. Such methods and devices have several unexpected advantages. First, as already described the second amplification step allows much faster thermocycling due to the smaller reaction volume. Secondly, also in case the nucleic acids of the sample are still partially bound to the binding matrix used for sample preparation and are not completely eluted from said matrix these nucleic acids can still be amplified because the binding matrix is present during the first number of thermocycles. And thirdly, in case said binding matrix inhibits the amplification reaction to some extend this inhibition effect is no longer present when subjecting the reaction to the second number of thermocycles, because the aliquot of the first reaction mixture used for conducting the second number of thermocycles is no longer in contact with said binding matrix.

A thermocycle is defined as a sequence of at least two temperatures, which the reaction mixture is subjected to for defined periods of time. This thermocycle can be repeated. In PCR methods usually three different temperatures are used. At around 45 - 70 °C the primers are annealed to the target nucleic acids. At a temperature at around 72 °C the primers bound to the target are elongated by a thermostable polymerase and subsequently at around 90 - 100°C, the double-stranded nucleic acids are being separated. In the PCR method, this thermocycle is usually repeated around 30 to 50 times. The time necessary for changing the temperature within the reaction mixture mainly depends on the volume and the shape of the reaction vessel and usually varies from several minutes down to a fraction of a second.

Prior to subjecting a partial amount of the first reaction mixture to a second number of thermocycles, it is preferred to transfer this partial amount of the reaction mixture to a second amplification chamber. This can be done manually by using a pipette. However, in view of the contamination risk, it is preferred if this is being automated in the device for example by pumps and valves. The first and second amplification chamber can be separated from each other by channels, valves, hydrophobic barriers and other means. Technical means for such integrated devices are known to an expert (see for example Lee et al., J. Micromech. Microeng. 13 (2003) 89-97; Handique et al., *Anal. Chem.* 72 4100-9; Hosokawa et al., *Anal. Chem.* 71 4781-5, Puntambekar et al., *Proc. Transducers'01* (Berlin: Springer) pp 1240-3, Zhao et al., *Science* 291 1023-6; Andersson et al., *Sensors Actuators* B 75 136-41)

It is also an option that the first and second amplification reaction chambers are two compartments in one unseparated reaction chamber without physical separation of both reaction mixtures. However, in this case it is necessary to avoid/minimize diffusion of the reaction products when conducting the second number of thermocycles, especially with regard to the amplified nucleic acids prepared within the first and second reaction compartment. This can be achieved by several means, for example by solid phase bound primers.

In case a channel is placed between the first and second amplification chamber physical separation by valves, vents, hydrophobic barriers can also be avoided in case the diffusion between both chambers is minimized.

The reaction mixture contains all ingredients necessary for conducting the amplification method of choice. Usually, these are primers allowing specific binding of the target nucleic acid to be amplified, enzymes like polymerases, reverse transcriptases and so on, nucleotide triphosphates, buffers, mono and divalent cations like magnesium. The ingredients depend on the amplification method and are well known to the expert.

The nucleic acid products prepared in the first and second reaction mixture can be detected by procedures known in the art, for example by detecting the length of the products in an agarose gel. By using sequence specific oligonucleotide probes, a further level of specificity can be achieved, for example by conducting a Southern or dot blot techniques. In homogenous amplification and detection methods, the detection probe or other detection means are already present in the reaction mixture during generation of the amplified nucleic acids. In the method described in EP 0 543 942 the probe is being degraded by the processing polymerase when elongating the primes. Usually well known labels can be used for detection. Examples are fluorescence labels like fluorescein, rhodamine and so on.

Therefore, one aspect of the present invention is directed to a method for determining the presence or absence or amount of a template nucleic acid in a sample comprising
- a): subjecting a first amount of said sample in a first amplification chamber to a first number of thermocycles to prepare a first amount of a reaction mixture,
- b): subjecting a partial amount of said first reaction mixture in a second amplification chamber to a second number of thermocycles to prepare a second amount of a second reaction mixture, and
- c): determining the formation of nucleic acids as a measure of the presence or absence or amount of nucleic acids to be determined
wherein the volume of said second amplification chamber preferably is smaller than the volume of said first amplification chamber. The integral heating and cooling speed preferably is at least 2 Kelvin/ second (K/s) in step a) and higher in step b), preferably at least 5 K/s.

The formation of nucleic acids can either be determined after completion of steps a) and b), or during the amplification steps a) and/ or b).

When transferring the partial amount of the first reaction mixture to the second amplification chamber, usually no further reaction components are added. This avoids any opening of the reaction chambers, at least when done automatically and avoids any contamination risk. However, for specific applications adding of further reagents might be useful. For example, it might be useful to add further primers when conducting a nested PCR protocol or an additional probe allowing detection of a certain amplification product. These reagents might be added by hand, but can be also stored in the reaction device in liquid or solid form prior to the reaction and mixed upon transfer of the partial amount of the first reaction mixture into the second amplification chamber. In a specific embodiment of the present invention, these reagents, especially primers and probes are bound to the solid phase.

As only a partial amount of the first reaction mixture is used for preparing the second reaction mixture, in principle multiple second reaction mixtures can be derived from the first reaction mixture. This allows subjecting more than one partial amount of the first reaction mixture to a second number of thermocycles and therefore allowing a multiplex reaction protocol. This can be in the simplest case a parallel reaction of the same mixture satisfying the results obtained in this method. In case different primers and/or probes are added to the partial amount of the first reaction mixture, a real multiplex detection method, for example for detecting different alleles of a target is possible.

A possible device for the methods of the present invention is described in Example 3. As already discussed above, the method of the present invention is not restricted to certain devices. It can be conducted by hand using commercially available thermocyclers like Applied Biosystems 9700'er system and the LightCycler (Roche Diagnostics). However, this method is especially suited for functionally integrated devices be based on technologies as for example described in Micro Total Analysis Systems, Proceedings uTAS'94, A van den Berg, P Berveld, 1994; Integrated Microfabricated Biodevices, M J Heller, A Guttman, 2002; microsystem Engineering of Lab-on-a-Chip devices, O Geschke, H Klank, P Tellemann, 2004; US 2003/0152492 and US 5,639,423. Such devices usually have an automated liquid transport, which allows transporting of a sample between reaction chambers, means for thermocycling, reagents which are either preloaded in the device or which can be added automatically, and means for detecting the reaction product. The reaction is being controlled by computer means and a computer program for controlling.

Therefore, another aspect of the present invention is a diagnostic device for preparing nucleic acids from a template comprising
- a first amplification chamber, and
- a second amplification chamber,
wherein the volume of said second amplification chamber preferably is smaller than the volume of said first amplification chamber. The integral heating and cooling speed preferably is at least 2 Kelvin/ second (K/s) in step a) and higher in step b), preferably at least 5 K/s.

The smaller size of the volume of the second amplification chamber allows decreasing the time necessary for each thermocycle. In standard thermocyclers, like the PCR System 9700 (Applied Biosystems) the size of the volume of the amplification chambers is not changed and, in addition most often metal blocks are used for thermocycling which does not allow to decrease the time necessary for a thermocycle to less than a few minutes. Therefore, taking an aliquot of an amplification reaction and using a faster thermocycler like the LightCycler for a second amplification reaction allows decreasing the overall reaction time without decreasing the sensitivity of the assay.

Amplification chambers suitable for a diagnostic device of the present invention basically are known in the prior art. These chambers do provide space for containing the reaction mixture. This chamber can be for example a thin-wall plastic tube which is fitted into a bore hole in the metal block of a thermocycler such as the Perkin Elmer 9700er instrument or the inner volume of the glass capillary which can be placed into the LightCycler instrument. The volume of the amplification chamber is defined by the maximal volume of a reaction mixture which can be used in the reaction.

The reaction mixtures can be heated by using for example heating elements like Peltier- or resistance-heating elements. For cooling active cooling elements or passive cooling elements, like heat sinks can be used. For conducting the heat and cool to the reaction mixture contained in the amplification chamber several means are known. In many conventional thermocyclers metal blocks containing the amplification chambers are used for providing the heat and cool to the reaction mixture. In the LightCycler format a hot air stream floating around the glass capillary provides this function.

The diagnostic device of the present invention has at least two amplification chambers as described above. These chambers can either be situated in one instrument or separated on two different instruments, whereby the transfer of an aliquot of the first reaction mixture to the second amplification chamber can be done by manual pipetting or, preferably, is being automated. The apparatus according to the invention has a receptacle to contain the device. It also comprises means heating and cooling the chambers and preferably also for controlling the temperature of the amplification cycles during the thermocycles, preferably a unit for controlling loaded with a computer program as described below.

Therefore, a further aspect of the present invention is a computer program for controlling a method for the preparation of nucleic acids from a template nucleic acid using thermocycles, characterized in that the computer program is set to apply a first number of thermocycles to the sample and subsequently a second number of thermocycles having a shorter cycling time on a different volume of a reaction mixture originating from the same sample. A more preferred aspect of the present invention is directed to a computer program for controlling the methods for preparation of nucleic acids as described above.

Such computer programs can be stored on physical storage mean, such as a diskette or a CD.

A further aspect of the present invention is an apparatus for preparing nucleic acids comprising
- a thermocycler, and
- a unit for controlling the thermocycler,
wherein the unit for controlling the thermocycler is loaded with computer program as described above. This thermocycler is preferably a diagnostic device as described above.

The present invention is further described in the following examples:

### Examples

### Example 1

### Optimized PCR protocol

For conducting a 100 µl PCR reaction in a cubic reaction chamber the question has been raised: How many cycles in an optimized Aliquot PCR method shall be conducted in the 100 µl volume and after what number of cycles an aliquot of which size should be added to the second reaction chamber to conduct the method in a minimum of time without changing the limit of detection and loosing sensitivity. A typical PCR cycle in a 100 µl volume needs about 130 seconds. In an optimal PCR reaction the amount of amplified nucleic acid is about to be doubled per cycle. Therefore, after n cycles 1/2ⁿ of the volume of the first reaction can be used as partial amount being subjected to a second number of thermocycles, which can be cycled much faster due to the smaller volume. The exact time needed for the shortened thermocycle is defined on one side by the temperature profile and on the other side by the thermal diffusion distance. For the actual calculation it has been assumed that the heated volume has a cubical shape and is in contact with the heat source/sink via a single wall. Most of the time during PCR will be consumed due to heat diffusion from this single wall through the water to reach a homogeneous temperature distribution. The thermal diffusion time scales with the second power of the side length of the cubical volume, therefore reducing the volume by a factor of two reduces the diffusion time by the factor of 2^{2/3}. Furthermore a typical run of 50 thermocycles has been assumed.

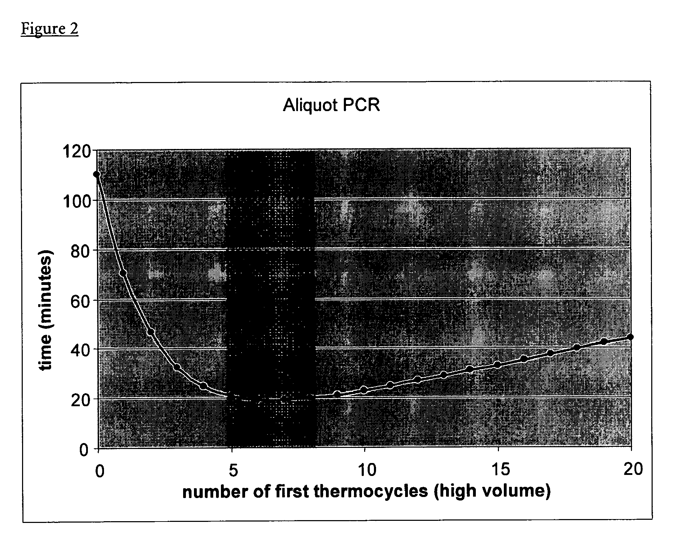

The result of this calculation is shown in Figure 2. In case 50 long thermocycles would be conducted, the reaction time would be 110 minutes. By applying the method of the present invention, this can be shorted to up to 20 minutes without losing sensitivity. As shown, it would be optimal to take an aliquot of the first reaction mixture after five to eight thermocycles and subject this partial amount to the remaining thermocycles, which can be performed faster due to the smaller volume. Depending on the number of the first thermocycles, 3.2 to 0.4 µl would be used for the second reaction. It should further be mentioned that reaction volumes of that size are well suited for detecting the amplified nucleic acid with standard detection methods like fluorescence detection.

Although this calculation is be based on some presumptions like doubling of the target nucleic acid per cycle (which is difficult to achieve in a real experiment), it very well illustrates the advantages of the present invention.

### Example 2

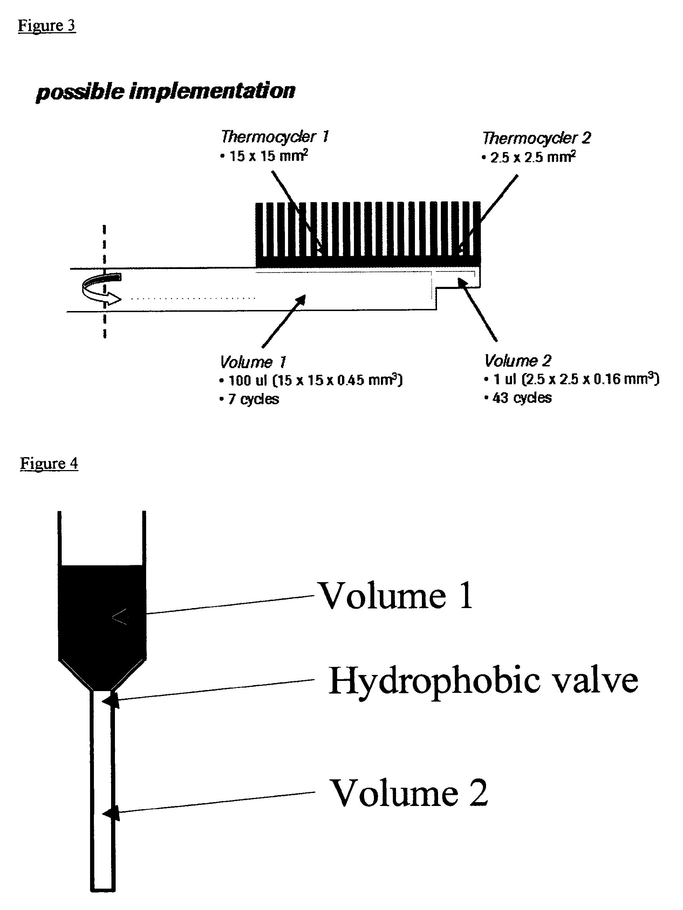

Figure 3 shows a scheme of a device having two amplification chambers and thermocycler elements, which is suitable for conducting the methods of the present invention. The two chambers are in physical contact via a narrow section which could be implemented as a hydrophobic valve. By this mean the second amplification chamber is not filled spontaneously when the first amplification chamber is being is filled. After several thermocycles, an aliquot of the first reaction mixture is being transferred to the second amplification chamber, for example by spinning the device or by applying hydrostatic pressure.

### Example 3

Figure 4 depicts a modification of a Light-Cycler® tube, characterized by narrow tube widening to the top of the tube. The wide section and the narrow section are separated from each other by a hydrophobic section (valve). After running the first few cycles in the upper half of the tube, an aliquot is spun down into the lower section of the tube allowing now much faster cycling profile. This of course requires some modification of the instrument to allow a centrifugation step within the cycling program. However this centrifugation step can also be conducted using available centrifuges without requiring modifications of the present Light-Cycler® device.

### Example 4

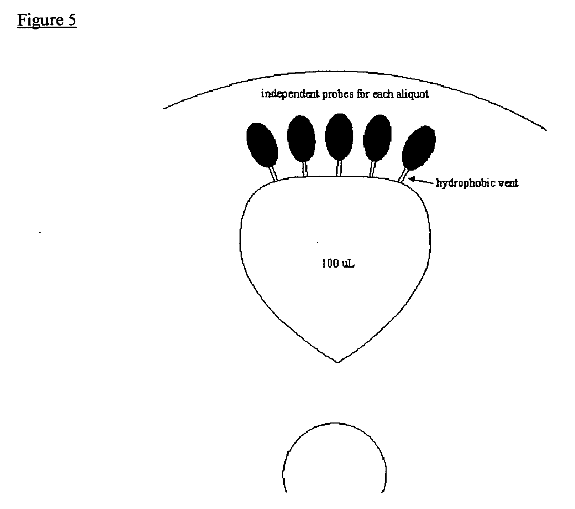

Figure 5 shows a scheme of a disk-shaped device having one reaction chamber for conducting the first number of thermocycles with a higher reaction volume and subjecting more than one partial amounts of that first reaction mixture to a second number of thermocycles, and, therefore allowing a multiplex reaction method. In this case the reaction liquid can be transported by spinning the disk device and applying centrifugal force, but also other methods like pneumatic force, vacuum and so on can be used. Usually it is advisable to reversibly block liquid connection between the first and second reaction chamber, for example by valves, hydrophobic vents and so on. However, as already outlined above, in case diffusion is minimized, it is also possible to use one reaction chamber having two reaction compartments, whereby the second compartment can be used for faster thermocycling. Minimization of diffusion of the amplified nucleic acids can be achieved for example by primers and/or probes being bound to the solid phase.

## Claims

1. A method of preparing nucleic acids from a template nucleic acid by subjecting a sample to thermocycles comprising
a) Subjecting a first amount of said sample in a first amplification chamber to a first number of thermocycles to prepare a first amount of a first reaction mixture, and
b) Subjecting a partial amount of said first reaction mixture in a second amplification chamber to a second number of thermocycles to prepare a second amount of a second reaction mixture
wherein the volume of said second amplification chamber is smaller than the volume of said first amplification chamber.

2. A method of preparing nucleic acids from a template nucleic acid by subjecting a sample to thermocycles comprising
a) Subjecting a first amount of said sample in a first amplification chamber to a first number of thermocycles to prepare a first amount of a first reaction mixture with an integral heating and cooling speed of at least 2 Kelvin/ second (K/s), and
b) Subjecting a partial amount of said first reaction mixture in a second amplification chamber to a second number of thermocycles to prepare a second amount of a second reaction mixture with an integral heating and cooling speed which is higher than that of said first amplification chamber and which is at least 5 K/s.

3. The method of claim 2 wherein the volume of said second amplification chamber is smaller than the volume of said first amplification chamber.

4. The method of any of claims 1 to 3 wherein the integral heating and cooling speed in step a) is 4 to 7 K/s and in step b) 8 to 12 K/s

5. The method of any of claims 1 to 4 wherein the volume of said first amount of said sample in said first amplification chamber has a volume of 5 to 200 µl, preferably 5 to 50 µl.

6. The method of any of claims 1 to 5 wherein the volume of said partial amount of said first reaction mixture has a volume of 0.05 to 5 µl, preferably 0.1 to 2 µl.

7. The method of any of claims 1 to 6 wherein said first number of thermocycles is smaller than the second number of thermocycles.

8. The method of any of claims 1 to 7, wherein the partial amount of the first reaction mixture is physically removed from the remainder of said first reaction mixture.

9. The method of claim 8, wherein the partial amount of the first reaction mixture is automatically removed from the remainder of said first reaction mixture by the device.

10. The method of any of claims 1 to 9, wherein the time used for a thermocycle in step b) is shorter than the time used for a thermocycle in step a).

11. The method of any of claims 1 to 10, wherein one or more additional partial amounts of said first reaction mixture are subjected to thermocycles in step b).

12. The method of any of claims 1 to 11, wherein a partial amount of said second reaction mixture is subjected to a third partial amount of thermocycles.

13. The method of any of claims 1 to 12, wherein the first amplification chamber is used for purification of the nucleic acids present in the unpurified sample prior to conducting said first number of thermocycles.

14. A method for determining the presence or absence or amount of a template nucleic acid comprising:
a) Subjecting a first amount of said sample in a first amplification chamber to a first number of thermocycles to prepare a first amount of a first reaction mixture, and
b) Subjecting a partial amount of said first reaction mixture in a second amplification chamber to a second number of thermocycles to prepare a second amount of a second reaction mixture, and
c) Determining the formation of nucleic acids as a measure of the presence or absence or amount of nucleic acids to be determined,
wherein the volume of said second amplification chamber is smaller than the volume of said first amplification chamber.

15. A method of preparing nucleic acids from a template nucleic acid by subjecting a sample to thermocycles comprising
a) Subjecting a first amount of said sample in a first amplification chamber to a first number of thermocycles to prepare a first amount of a first reaction mixture with an integral heating and cooling speed of at least 2 Kelvin/ second (K/s), and
b) Subjecting a partial amount of said first reaction mixture in a second amplification chamber to a second number of thermocycles to prepare a second amount of a second reaction mixture with an integral heating and cooling speed which is higher than that of said first amplification chamber and which is at least 5 K/s.
c) Determining the formation of nucleic acids as a measure of the presence or absence or amount of nucleic acids to be determined,

16. The method of claim 15 wherein the volume of said second amplification chamber is smaller than the volume of said first amplification chamber.

17. The method of any of claims 14 to 16, wherein the formation of nucleic acids is being determined (step c) after completion of steps a) and b).

18. The method of claim 13, wherein the formation of nucleic acids is being determined (step c) during step a) and/ or step b).

19. The method of any of claims 14 to 18 wherein the integral heating and cooling speed in step a) is 4 to 7 K/s and in step b) 8 to 12 K/s

20. The method of any of claims 14 to 19 wherein the volume of said first amount of said sample in said first amplification chamber has a volume of 5 to 200 µl, preferably 5 to 50 µl.

21. The method of any of claims 14 to 20 wherein the volume of said partial amount of said first reaction mixture has a volume of 0.05 to 5 µl, preferably 0.1 to 2 µl.

22. The method of any of claims 14 to 21 wherein said first number of thermocycles is smaller than the second number of thermocycles.

23. The method of any of claims 14 to 22, wherein the partial amount of the first reaction mixture is physically removed from the remainder of said first reaction mixture.

24. The method of claim 23, wherein the partial amount of the first reaction mixture is automatically removed from the remainder of said first reaction mixture by the device.

25. A diagnostic device for preparing nucleic acids from a template comprising
a. A first amplification chamber, and
b. A second amplification chamber,
wherein the volume of said second amplification chamber is smaller than the volume of said first amplification chamber.

26. A diagnostic device for preparing nucleic acids from a template comprising
a. A first amplification chamber having an integral heating and cooling speed of at least 2 K/s, and
b. A second amplification chamber having an integral heating and cooling speed which is higher than that of said first amplification chamber and which is at least 5 K/s,

27. The device of claim 26 wherein the volume of said second amplification chamber is smaller than the volume of said first amplification chamber.

28. The device of any of claims 25 to 27, wherein the integral heating and cooling speed of said first amplification chamber is 4 to 7 K/s and of said second amplification chamber is 8 to 12 K/s.

29. The device of any of claims 25 to 28, wherein the volume of said first amount of said first amplification chamber has a volume of 5 to 200 µl, preferably 5 to 50 µl.

30. The device of any of claims 25 to 29, wherein the volume of said second amplification chamber has a volume of 0.05 to 5 µl, preferably 0.1 to 2 µl.

31. The device of any of claims 25 to 30, wherein the first amplification chamber also allows purification of nucleic acids present in a sample prior to amplification.

32. The device of any of claims 25 to 31 having means for transporting liquids from said first amplification chamber to said second amplification chamber.

33. A computer program for controlling a method for the preparation of nucleic acids from a template nucleic acid using thermocycles, **characterized in that** the computer program is set to apply a first number of thermocycles to the sample and subsequently a second number of thermocycles having a shorter cycling time on a different volume of a reaction mixture originating from the same sample.

34. A computer program, optionally according to claim 33 for controlling a method of any of claims 1 to 24.

35. A computer program product comprising a program according to claim 33 or 34 on a physical storage means.

36. An apparatus for preparing nucleic acids comprising
a. a diagnostic device according to any of claims 25 to 32 and
b. a unit for controlling the diagnostic device,
wherein the unit for controlling the diagnostic device is loaded with a computer program according to any of claim 33 to 35.
